# EUROPÄISCHE PATENTSCHRIFT

(11) **EP 1 774 925 B1**
(45) Veröffentlichungstag und Bekanntmachung des Hinweises auf die Patenterteilung: **23.07.2008**
(21) Anmeldenummer: 06021122.4
(22) Anmeldetag: 09.10.2006
(51) Int. Cl.: A61F 2/18

(54) **Gehörknöchelchenprothese mit elastischem Drehgelenk**
Auditory ossicles prosthesis with elastic swivel joint
Prothèse d'osselets de l'oreille à joint articulé élastique

(30) Priorität: 11.10.2005 DE 102005048618
(43) Veröffentlichungstag der Anmeldung: 18.04.2007
(73) Patentinhaber: Heinz Kurz GmbH Medizintechnik, 72144 Dusslingen (DE)
(72) Erfinder: Steinhardt, Uwe, 72145 Hirrlingen (DE); Kurz, Heinz, 72144 Dusslingen (DE)
(74) Vertreter: Kohler Schmid Möbus

(56) Entgegenhaltungen:
- EP-A- 1 094 687
- EP-A2- 0 901 779
- DD-A1- 259 566
- DE-A1- 10 045 158
- DE-U1-2202005 003 78
- US-A- 4 281 419
- US-A- 4 601 723

## Beschreibung

Die Erfindung betrifft eine Gehörknöchelchenprothese, die mindestens ein Glied oder Teile eines Gliedes der Gehörknöchelchenkette ersetzt oder überbrückt, wobei die Gehörknöchelchenprothese an ihrem einen Ende ein als Kopfplatte zur mechanischen Verbindung mit dem Trommelfell ausgebildetes erstes Befestigungselement und an ihrem anderen Ende ein zweites Befestigungselement zur mechanischen Verbindung mit einem Glied oder Teilen eines Gliedes der Gehörknöchelchenkette oder mit dem Innenohr sowie zwischen den beiden Befestigungselementen ein Drehgelenk aufweist, das ein aus einem elastischen Kunststoff gefertigtes Aufnahmeteil umfasst, welches starr mit dem ersten Befestigungselement verbunden ist, in welchem ein Drehelement, das fest mit dem zum ersten Befestigungselement gerichteten Ende eines länglichen Schafts verbunden ist, der die beiden Befestigungselemente miteinander verbindet, gelenkig gelagert ist, und welches einen Hohlraum aufweist, welcher derart geformt ist, dass er im montierten Zustand mindestens die halbe Oberfläche des Drehelements einhüllend umschließt.

Eine derartige Vorrichtung ist bekannt aus der US-A-4 281 419, welche als nächstliegender Stand der Technik angesehen ist.
Gehörknöchelchenprothesen werden verwendet, um bei ganz oder teilweise fehlenden oder geschädigten Gehörknöchelchen des menschlichen Mittelohrs den Schall vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese beispielsweise mittels einer Kopfplatte am Ambossfortsatz der menschlichen Gehörknöchelchenkette befestigt und das andere Ende der Gehörknöchelchenprothese beispielsweise am Steigbügel der menschlichen Gehörknöchelchenkette befestigt oder direkt ins Innenohr getaucht wird. Vielfach wird mit den bekannten Gehörknöchelchenprothesen die Schallleitung zwischen dem Trommelfell und dem Innenohr nur begrenzt ermöglicht, weil sie die natürlichen anatomischen Ausbildungen der Gehörknöchelchenkette nur sehr eingeschränkt ersetzen können.

Nachdem die Prothese operativ im Mittelohr platziert wurde und das Trommelfell wieder verschlossen ist, beginnt die so genannte Einheilphase. In dieser Zeit bilden sich Narben und diese verursachen unvorhersehbar Kräfte, welche dazu führen können, die Prothese aus ihrer lokalen Position zu verschieben. Bei einer steifen Verbindung zwischen Kopfplatte und Schaft kann es zwischen der Kante der Kopfplatte und dem Trommelfell bzw. dem Transplantat zwischen Trommelfell und Kopfplatte zu erhöhten Druckspitzen kommen. Diese können so hoch sein, dass eine Penetration durch das Trommelfell die Folge wäre. Aus diesem Grund ist es sehr hilfreich, wenn die Prothese eine gewisse post-operative Mobilität aufweist, so dass sich die Kopfplatte postoperativ selbstständig der Position des Trommelfells angleichen kann.

Da zudem die anatomischen Gegebenheiten des Ohrs, wie beispielsweise die Lage, die Form und die Größe des Steigbügels, des Ambosses, des Hammers und des Trommelfells variieren, ist es sehr vorteilhaft, wenn Gehörknöchelchenprothesen nicht starr ausgebildet sind, sondern eine gewisse Flexibilität oder Variabilität aufweisen.

Um diese Flexibilität/Variabilität zu erreichen sind verschiedene Befestigungs- und Ankopplungsvorrichtungen für Gehörknöchelchen, die elastische Teile und/oder Gelenke aufweisen, bekannt. Eine solche gelenkige Verbindung zwischen einem an der Steigbügelfußplatte montierbaren Befestigungselement und dem länglichen Schaft ist beispielsweise in der EP 1 181 907 B1 beschrieben und wird von der Anmelderin unter dem Markennamen "Ball-Joint" angeboten.

Aufgrund der anatomisch- und abstammungsbedingten Variationsbreite werden im Rahmen einer Tympanoplastik unterschiedlich lange Mittelohrprothesen für die Rekonstruktion der Gehörknöchelchenkette in der Otologie benötigt. Leider ist es derzeit vor einer Operation nicht möglich, die tatsächlich erforderliche Länge im Voraus zu wissen. Deshalb muss man entweder ein umfangreiches Sortiment von verschieden langen Prothesen auf Lager halten, was mit hohen Kosten verbunden ist, oder die Prothese muss in ihrer Länge variabel sein, so dass sie unmittelbar vor ihrer Implantation individuell auf den jeweiligen Patienten abgestimmt werden kann. Da auch die relative Lage der Gehörknöchelchenprothese zum Trommelfell bei jedem Menschen etwas differiert, muss das Mittelohr vor dem Einsatz der Prothese hinsichtlich der Lage der Befestigungselemente genau vermessen werden. Die erforderliche Länge des Schaftes kann aber erst während der Operation bestimmt werden, so dass also für jede Operation entweder ein Satz von Prothesen unterschiedlicher Länge oder eine Prothese mit variabler Schaftlänge bereitgestellt werden muss.

Eine günstige Lösung, bei der das Ablängen des Schaftes auf die individuelle Länge mit einem geringen Fertigungsaufwand für den Schaft und damit kostengünstiger folgen kann, ist beispielsweise in der EP 0 998 884 B1 beschrieben. Derartige Gehörknöchelchenprothesen werden von der Anmelderin unter dem Markennamen "VARIO" angeboten. Bei diesen bekannten Prothesen ragt der Schaft durch eine Durchgangsbohrung des als Kopfplatte ausgebildeten ersten Befestigungselements hindurch. Der Schaft kann durch die Durchgangsbohrung axial so verschoben werden, dass er auf der Außenseite der Kopfplatte übersteht und dort abgelängt werden kann, wobei die Durchgangsbohrung hinterher verengbar ist, um den Schaft an der gewünschten Stelle zu fixieren.

Eine ähnliche Technik hinsichtlich der Ablängung ist auch in der US 6,168,625 B1 bzw. der DE 100 45 158 A1 beschrieben, wobei hier der wellenartig gefertigte Schaft längs seiner Achse eine Vielzahl von Einkerbungen aufweist, die als Sollbruchstellen für die spätere unproblematische Ablängung während der Operation dienen sollen.

Allerdings weisen die letztgenannten Gehörknöchelchenprothesen wiederum nicht die oben beschriebenen Gelenkstellen zur Erzeugung einer post-operativen Mobilität auf. Demgegenüber verbindet die in der DE 20 2005 003 782 U1 beschriebene Gehörknöchelchenprothese, bei der der Schaft eine Vielzahl von aneinander angrenzenden Kugeln umfasst, die eine Kugelkette bilden und während der Operation leicht auf eine gewünscht Länge gekürzt werden kann, die Vorteile beider Arten von Prothesen. Nachteilig bei dieser bekannten Gehörknöchelchenprothese ist allerdings die Art und der Aufbau des Drehgelenks, bei welchem das Aufnahmeteil für das Drehelement aus zwei parallel verlaufenden und einen spaltförmigen Raum zwischen sich einschließenden Metallstegen besteht, in dem eine Kugel, die Teil des länglichen Schafts ist, in zwei kreisförmigen Ausnehmungen der Stege gelenkig gelagert ist. Dabei ragen die beiden Metallstege in eine dafür vorgesehenen Durchbrechung des ersten Befestigungselements und sind lateral beweglich und biegbar, so dass der Schaft im montierten Zustand des Drehgelenks quer zu seiner Achsrichtung in undefinierter Weise ausweichen kann und keine gute schallharte Verbindung zwischen dem ersten und dem zweiten Befestigungselement gewährleistet ist, so dass keine optimale Schallleitung durch die Prothese gesichert ist.

Demgegenüber besitzen gattungsgemäße Gehörknöchelchenprothesen, wie sie etwa in der eingangs zitierten US-A-4 281 419 beschrieben sind, zwar aufgrund des Kugelgelenks an der Kopfplatte eine gewisse post-operative Mobilität, so dass sich die Kopfplatte nach der Operation selbständig der aktuellen Position des Trommelfells in gewissen Grenzen angleichen kann. Nachteilig bei dieser Klasse von bekannten Gehörknöchelchenprothesen ist jedoch die fehlende Längenvariabilität. Insbesondere ist es bei den aus der US-A-4 281 419 bekannten Prothesen absolut unmöglich, während der Operation die benötigte Länge zwischen den beiden Ankoppelpunkten exakt einzustellen.

Aufgabe der vorliegenden Erfindung ist es daher, eine gattungsgemäße Vorrichtung der eingangs beschriebenen Art dahin gehend zu verbessern, dass einerseits die Gelenkigkeit des Schafts der Prothese in der Kopfplatte in einer relativ schallharten Weise erhalten bleibt, dass aber andererseits eine problemlose Längenvariabilität der Prothese währende der Operation ermöglicht wird.

Erfindungsgemäß wird diese Aufgabe auf ebenso überraschend einfache wie wirkungsvolle Art und Weise dadurch gelöst, dass der längliches Schaft eine Vielzahl von aneinander angrenzenden weiteren Drehelementen umfasst, und dass der Hohlraum des Aufnahmeteils zylindrisch geformt ist, im montierten Zustand der Prothese das Drehelement ringförmig umgibt und eine leichte Pressung auf das Drehelement ausübt, wobei der zylindrische Hohlraum eine Durchgangsöffnung im Aufnahmeteil bildet, durch die die Drehelemente vor oder während der Implantation der Prothese in Richtung auf die Anlageseite der Kopfplatte am Trommelfell durchgesteckt und die überstehenden Drehelemente ab dem weiteren Drehelement dann zur Ablängung des Schafts abgezwickt werden können.

Dadurch werden auf simple Weise die Vorteile der oben beschriebenen bekannten Gehörknöchelchenprothese gemäß der gattungsbildenden US-A-4 281 419 genutzt, die eine relativ schallharte Aufbauweise des Drehgelenks und damit eine optimale Schallleitung durch die Prothese ermöglicht. Dies wird dadurch sichergestellt, dass das Aufnahmeteil das Drehelement - anders als beim Kugelgelenk nach der DE 20 2005 003 782 U1 - in einem großen Oberflächenbereich fest und relativ starr umschließt, wobei der umhüllte Bereich bei Ausführungsformen der Erfindung auch etwas weniger, in anderen Ausführungsformen aber auch mehr als Hälfte der Oberfläche des Drehelements betragen kann. Die Verwendung von elastischem Kunststoff für das Aufnahmeteil gewährleistet andererseits trotzdem die gewünschte post-operative Mobilität und erleichtert die Montage des Drehelements, wobei aber die spezielle Geometrie des Aufnahmeteils und des Drehelements nach der Montage ein undefiniertes Ausweichen der Anordnung verhindert und nur Drehgelenkbewegungen in einem festlegbaren Umfang zulässt.

Sehr wichtig ist nämlich auch die selbstständige postoperative Krafteinstellung im Lager des Drehgelenks. Zum einen darf sie nicht zu hoch sein, denn dann findet keine Bewegung statt; zum anderen darf sie nicht zu niedrig sein, denn dies könnte zu einer fehlerhaften Signalübertragung führen. Mit der erfindungsgemäßen Vorrichtung wird auch hierfür eine optimale Lösung bereitgestellt.

In diesem Zusammenhang ist eine Ausführungsform der Erfindung besonders vorteilhaft, bei der das elastische Material des Aufnahmeteils so gewählt ist, dass seine Oberfläche bei Raumtemperatur gute Gleiteigenschaften aufweist. Dadurch können auf einfache Weise störende Reibungseinflüsse bei der Gelenkbewegung minimiert werden.

Fertigungstechnisch relativ einfach realisierbar ist eine Ausführungsform, bei der das Drehelement als Kugel oder Teil einer Kugel, insbesondere als Halbkugel ausgeführt ist.

Eine vorteilhafte Weiterbildung dieser Ausführungsform sieht vor, dass das Drehelement als Rotationsellipsoid oder Teil eines Rotationsellipsoids ausgeführt ist, was zwar etwas mehr Aufwand in der Herstellung erfordert als ein Kugelgelenkteil, aber dafür Vorteile in der Beweglichkeit des Gelenks bietet.

Eine weitere bevorzugte Ausführungsform der Erfindung umfasst eine Gehörknöchelchenprothese, bei der das Aufnahmeteil in das erste Befestigungselement eingepasst, insbesondere eingepresst ist.

Alternativ oder zusätzlich zur Erzielung eines besonders sicheren Haltes kann bei anderen Ausführungsformen der erfindungsgemäßen Gehörknöchelchenprothese das Aufnahmeteil mit dem ersten Befestigungselement verklebt oder verschweißt sein.

Besonders kompakt und daher preiswert herstellbar ist eine dazu alternative Ausführungsform der erfindungsgemäßen Gehörknöchelchenprothese, bei der das Aufnahmeteil integraler Bestandteil des als Kopfplatte ausgeführten ersten Befestigungselements ist.

Bei bevorzugten Ausführungsformen werden das Aufnahmeteil der erfindungsgemäßen Gehörknöchelchenprothese aus Silikon oder PTFE und das Drehelement sowie der längliche Schaft aus Metall gefertigt sein.

Besonders einfach und preiswert in der Herstellung ist eine Ausführungsform, bei der die aneinander angrenzenden weiteren Drehelemente des länglichen Schafts identisch wie das Drehelement geformt sind, insbesondere jeweils den gleichen Außendurchmesser aufweisen, und äquidistant längs der Achse des Schafts angeordnet sind. Dies erleichtert auch die Handhabung beim Ablängen des Schaftes während der operativen Implantation.

Je nach dem individuellen Defekt, der bei einem Patienten durch den Einsatz der erfindungsgemäßen Gehörknöchelchenprothese behoben oder zumindest in seinen Auswirkungen gelindert werden soll, wird der Aufbau der Prothese entsprechend gestaltet sein. Bei sämtlichen Ausführungsformen der Erfindung wird das erste Befestigungselement eine zur Anlage am Trommelfell ausgebildete Kopfplatte umfassen.

Eine Klasse von Ausführungsformen der erfindungsgemäßen Vorrichtung zeichnet sich dadurch aus, dass das zweite Befestigungselement plattenförmig, glockenförmig, hülsenförmig, stempelförmig oder als Clip ausgebildet ist.

Bei Weiterbildungen dieser Ausführungsformen ist die Gehörknöchelchenprothese über die Kopfplatte einerseits am Trommelfell und über das zweite Befestigungselement andererseits am Amboss oder am Steigbügel befestigt.

Alternative Ausgestaltungen können vorsehen, dass die Gehörknöchelchenprothese an ihrem das zweite Befestigungselement tragenden Ende mittels Eröffnung der menschlichen Hörschnecke (=Cochleotomie) direkt an das Innenohr angekoppelt ist, insbesondere über einen Kolben.

Bevorzugt ist eine Ausführungsform der erfindungsgemäßen Vorrichtung, bei der die Prothese oder Teile davon aus biokompatiblen Kunststoffen, insbesondere Silikon, oder Faserverbundwerkstoffen hergestellt ist. Dadurch können postoperative Abstoßungsreaktionen in den meisten Fällen verhindert werden.

Die erfindungsgemäße Gehörknöchelchenprothese oder Teile davon können aus Titan und/oder aus Gold und/oder aus Tantal und/oder aus einer Legierung der genannten Metalle hergestellt sein.

Vorteilhaft im Hinblick auf die oben erwähnte postoperative Lageanpassung sind Ausführungsformen der Erfindung, bei denen die Prothese oder Teile davon aus einem Material mit Formgedächtnis (=memory effect), insbesondere aus Nitinol hergestellt sind.

Die Massenverteilung der einzelnen Teile der Prothese kann in Abhängigkeit von einem gewünschten, vorgebbaren Frequenzgang der Schallleitung im Mittelohr berechnet werden. Damit lässt sich gewissermaßen ein Tuning der Schallfortpflanzungs-Eigenschaften mittels einer individuellen ausgestalteten Gehörknöchelchenprothese erreichen. Ein solcher Tuning Effekt kann bei speziellen Ausführungsformen beispielsweise dadurch erzielt werden, dass mindestens eine zusätzliche Masse in Abhängigkeit von einem gewünschten, vorgebbaren Frequenzgang der Schallleitung im Mittelohr an einem Teil der Gehörknöchelchenkette bzw. der Prothese befestigt ist.

Bei vorteilhaften Weiterbildungen dieser Ausführungsformen ist die zusätzliche Masse mittels eines Clips an einem Teil der Gehörknöchelchenkette oder der Prothese befestigt.

Eine weitere Ausführungsform der Erfindung schließlich zeichnet sich dadurch aus, dass die Prothese mit einem aktiven Vibrationsteil eines aktiven, insbesondere implantierbaren Hörgeräts verbunden ist. Damit lassen sich auch weitergehende Gehörschäden durch Einsatz moderner Elektronik in weiten Bereichen beheben oder zumindest in ihren Auswirkungen wesentlich lindern.

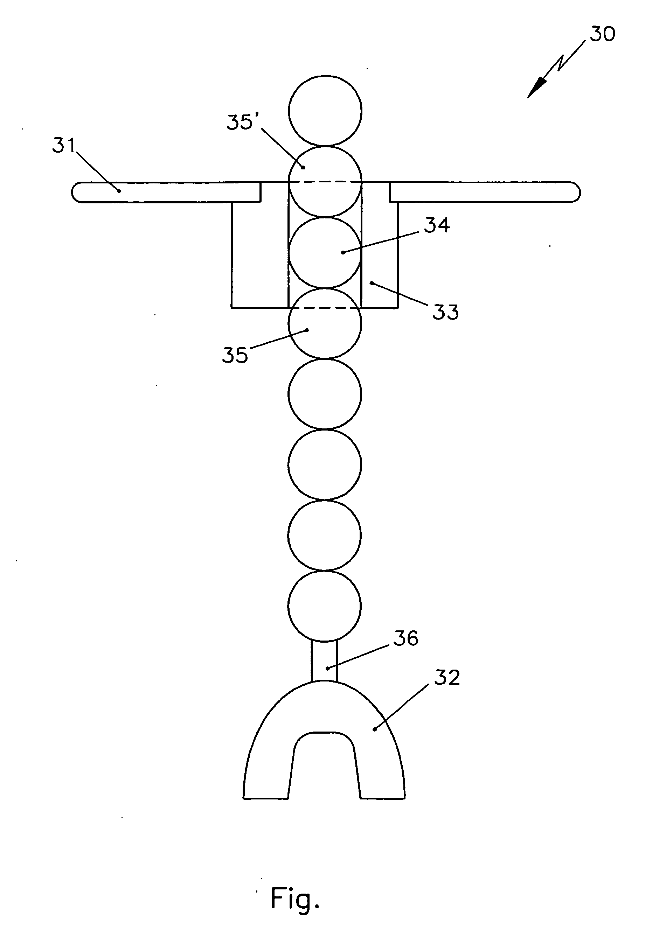

Weitere Merkmale und Vorteile der Erfindung ergeben sich aus der nachfolgenden detaillierten Beschreibung eines Ausführungsbeispiels der Erfindung anhand der Figur der Zeichnung, die erfindungswesentliche, Einzelheiten zeigt, sowie aus den Ansprüchen. Die einzelnen Merkmale können je einzeln für sich oder zu mehreren in beliebigen Kombinationen bei Varianten der Erfindung verwirklicht sein.

In der schematischen Zeichnung ist ein Ausführungsbeispiel der Erfindung dargestellt, welches in der nachfolgenden Beschreibung näher erläutert wird.

Die Figur zeigt einen schematischen Längsschnitt durch eine Ausführungsform der erfindungsgemäßen Gehörknöchelchenprothese mit einem als Kopfplatte ausgebildeten ersten Befestigungselement und einem glockenförmigen zweiten Befestigungselement am anderen Ende des länglichen. Schaftes sowie mit einer Kette von kugelförmigen Drehelementen am Schaft und Durchführung durch das Aufnahmeteil.

Die in der Figur sehr schematisch im Längsschnitt dargestellte Gehörknöchelchenprothese 30 weist ein erstes Befestigungselement 31 auf, welches in Form einer Kopfplatte zur Anlage am Trommelfell ausgebildet ist. Am anderen Ende der Gehörknöchelchenprothese 30 ist ein zweites Befestigungselement 32 vorgesehen, das bei dem vorliegenden Ausführungsbeispiel Eine glockenartige Gestaltung aufweist und zur Befestigung der Gehörknöchelchenprothese 30 an einem Glied der Gehörknöchelchenkette, beispielsweise am Amboss oder am Steigbügel dient.

Das erste Befestigungselement 31 ist mit dem zweiten Befestigungselement 32 durch einen länglichen Schaft 36 verbunden, der eine Vielzahl von aneinander angrenzenden weiteren Drehelementen 35, 35' umfasst. Die äquidistant längs der Achse des Schafts 36 angeordneten Drehelemente 34, 35, 35' sind als Kugeln gleicher Größe ausgeführt. Zur Ablängung können diese Kugeln durch eine Durchgangsöffnung im Aufnahmeteil 33 in Richtung auf die Anlageseite der Kopfplatte 31 am Trommelfell durchgesteckt und die überstehenden Kugeln ab dem weiteren Drehelement 35' dann einfach abgezwickt werden. An seinem entgegengesetzten Ende mündet der Schaft 36 bei dieser Ausführungsform wieder in ein glockenförmiges zweites Befestigungselement 32.

Das aus einem elastischen Kunststoff gefertigte Aufnahmeteil 33 wiederum ist starr mit dem ersten Befestigungselement 31 verbunden, und zwar in der in der Figur gezeigten Ausführungsform durch Einpressung eines zylindrischen Abschnitts an der Oberseite des Aufnahmeteils 33 in eine entsprechende kreisrunde Bohrung im Zentrum des ersten Befestigungselements 31. Zusätzlich oder alternativ kann auch eine Verklebung oder Verschweißung des zylindrischen Abschnitts mit der kreisrunden Bohrung erfolgen. Vorzugsweise ist das elastische Material des Aufnahmeteils 33 so gewählt, dass seine Oberfläche bei Raum- bzw. Körpertemperatur gute Gleiteigenschaften aufweist, insbesondere Silikon oder PTFE, so dass die Beweglichkeit der Drehelemente 34, 35, 35' im Aufnahmeteil 33 durch den geringen Reibungswiderstand besonders hoch ist.

Das Drehelement 34 bildet zusammen mit dem Aufnahmeteil 33 ein Drehgelenk, das der erfindungsgemäßen Gehörknöchelchenprothese 30 einerseits eine post-operative Beweglichkeit, andererseits aber durch die starre, relativ schallharte Anbindung des Drehgelenks an die beiden Befestigungselemente 31, 32 gute Schallleitungseigenschaften verleiht.

Die Massenverteilung der einzelnen Teile der erfindungsgemäßen Prothese kann in Abhängigkeit von einem gewünschten, vorgebbaren Frequenzgang der Schallleitung im Mittelohr berechnet sein, um ein individuelles Tuning der Schallleitungseigenschaften zu ermöglichen.

## Patentansprüche

1. Gehörknöchelchenprothese (30), die mindestens ein Glied oder Teile eines Gliedes der Gehörknöchelchenkette ersetzt oder überbrückt, wobei die Gehörknöchelchenprothese (30) an ihrem einen Ende ein als Kopfplatte zur mechanischen Verbindung mit dem Trommelfell ausgebildetes erstes Befestigungselement (31) und an ihrem anderen Ende ein zweites Befestigungselement (32) zur mechanischen Verbindung mit einem Glied oder Teilen eines Gliedes der Gehörknöchelchenkette oder mit dem Innenohr sowie zwischen den beiden Befestigungselementen (31,32) ein Drehgelenk aufweist, das ein aus einem elastischen Kunststoff gefertigtes Aufnahmeteil (33) umfasst, welches starr mit dem ersten Befestigungselement (31) verbunden ist, in welchem ein Drehelement (34), das fest mit dem zum ersten Befestigungselement (31) gerichteten Ende eines länglichen Schafts (36) verbunden ist, der die beiden Befestigungselemente (31,32) miteinander verbindet, gelenkig gelagert ist, und welches einen Hohlraum aufweist, welcher derart geformt ist, dass er im montierten Zustand mindestens die halbe Oberfläche des Drehelements (34) einhüllend umschließt,
**dadurch gekennzeichnet,**
**dass** der längliche Schaft (36) eine Vielzahl von aneinander angrenzenden weiteren Drehelementen (35, 35') umfasst, und dass der Hohlraum des Aufnahmeteils (33) zylindrisch geformt ist, im montierten Zustand der Prothese das Drehelement (34) ringförmig umgibt und eine leichte Pressung auf das Drehelement (34) ausübt, wobei der zylindrische Hohlraum eine Durchgangsöffnung im Aufnahmeteil (33) bildet, durch die die Drehelemente (34, 35, 35') vor oder während der Implantation der Prothese in Richtung auf die Anlageseite der Kopfplatte (31) am Trommelfell durchgesteckt und die überstehenden Drehelemente ab dem weiteren Drehelement (35') dann zur Ablängung des Schafts (36) abgezwickt werden können.

2. Gehörknöchelchenprothese nach Anspruch 1, **dadurch gekennzeichnet, dass** das elastische Material des Aufnahmeteils (13; 23; 33) so gewählt ist, dass seine Oberfläche bei Raum- bzw. Körpertemperatur gute Gleiteigenschaften aufweist.

3. Gehörknöchelchenprothese nach Anspruch 1 oder 2, **dadurch gekennzeichnet, dass** das Drehelement (34) als Kugel oder Teil einer Kugel, insbesondere als Halbkugel ausgeführt ist.

4. Gehörknöchelchenprothese nach Anspruch 1 oder 2, **dadurch gekennzeichnet, dass** das Drehelement (34) als Rotationsellipsoid oder Teil eines Rotationsellipsoids ausgeführt ist.

5. Gehörknöchelchenprothese nach einem der Ansprüche 1 bis 4, **dadurch gekennzeichnet, dass** das Aufnahmeteil (33) in das erste Befestigungselement (31) eingepasst, insbesondere eingepresst ist.

6. Gehörknöchelchenprothese nach einem der Ansprüche 1 bis 5, **dadurch gekennzeichnet, dass** das Aufnahmeteil (33) mit dem ersten Befestigungselement (31) verklebt oder verschweißt ist.

7. Gehörknöchelchenprothese nach einem der Ansprüche 1 bis 4, **dadurch gekennzeichnet, dass** das Aufnahmeteil (33) Bestandteil des als Kopfplatte ausgeführten ersten Befestigungselements (31) ist.

8. Gehörknöchelchenprothese nach einem der vorhergehenden Ansprüche, **dadurch gekennzeichnet, dass** das Aufnahmeteil (33) aus Silikon oder PTFE gefertigt ist.

9. Gehörknöchelchenprothese nach einem der vorhergehenden Ansprüche, **dadurch gekennzeichnet, dass** das Drehelement (34) und der längliche Schaft (36) aus Metall gefertigt sind.

10. Gehörknöchelchenprothese nach einem der vorhergehenden Ansprüche, **dadurch gekennzeichnet, dass** die aneinander angrenzenden weiteren Drehelemente (35, 35') des länglichen Schafts (36) identisch wie das Drehelement (34) geformt sind, insbesondere jeweils den gleichen Außendurchmesser aufweisen, und äquidistant längs der Achse des Schafts (36) angeordnet sind.

11. Gehörknöchelchenprothese nach einem der vorhergehenden Ansprüche, **dadurch gekennzeichnet, dass** das zweite Befestigungselement (32) plattenförmig, glockenförmig, hülsenförmig, stempelförmig oder als Clip ausgebildet ist.

12. Gehörknöchelchenprothese nach Anspruch 11, **dadurch gekennzeichnet, dass** die Gehörknöchelchenprothese (30) einerseits am Trommelfell und andererseits am Amboss oder am Steigbügel befestigbar ist.

13. Gehörknöchelchenprothese nach einem der Ansprüche 1 bis 10, **dadurch gekennzeichnet, dass** die Gehörknöchelchenprothese mittels Eröffnung der menschlichen Hörschnecke, was gleichbedeutend mit Cochleotomie ist, einenends direkt, insbesondere über einen Kolben an das Innenohr ankoppelbar ist.

14. Gehörknöchelchenprothese nach einem der vorhergehenden Ansprüche, **dadurch gekennzeichnet, dass** die gesamte Gehörknöchelchenprothese (30) oder Teile davon aus biokompatiblen Kunststoffen, insbesondere Silikon, und/oder aus Faserverbundwerkstoffen hergestellt sind.

15. Gehörknöchelchenprothese nach einem der vorhergehenden Ansprüche, **dadurch gekennzeichnet, dass** Teile der Gehörknöchelchenprothese (30) aus Titan und/oder aus Gold und/oder aus Tantal und/oder aus einer Legierung der genannten Metalle hergestellt sind.

16. Gehörknöchelchenprothese nach einem der vorhergehenden Ansprüche, **dadurch gekennzeichnet, dass** Teile der Gehörknöchelchenprothese (30) aus einem Material mit Formgedächtnis, was gleichbedeutend mit memory effect ist, insbesondere aus Nitinol hergestellt sind.

17. Gehörknöchelchenprothese nach einem der vorhergehenden Ansprüche, **dadurch gekennzeichnet, dass** mindestens eine zusätzliche Masse in Abhängigkeit von einem gewünschten, vorgebbaren Frequenzgang der Schallleitung im Mittelohr an der Gehörknöchelchenprothese oder an einem Teil der Gehörknöchelchenkette befestigt ist, insbesondere mittels eines Clips.

18. Gehörknöchelchenprothese nach einem der vorhergehenden Ansprüche, **dadurch gekennzeichnet, dass** die Prothese mit einem aktiven Vibrationsteil eines aktiven, insbesondere implantierbaren Hörgeräts verbunden ist.

## Claims

1. An auditory ossicle prosthesis (30) which replaces or bridges at least one link or parts of a link of the auditory ossicle chain, wherein the auditory ossicle prosthesis (30) has at its one end a first fastening element (31) designed as a head plate for mechanical connection to the eadrum, and has at its other end a second fastening element (32) for mechanical connection to a link or parts of a link of the auditory ossicle chain or to the inner, and wherein it has between the two fastening elements (31, 32) a hinge which comprises a receiving part (33) which is produced from an elastic plastic and which is connected rigidly to the first fastening element (31) in which is articulated a rotary element (34), which is fixedly connected to the end of an oblong shaft (36) directed towards the first fastening element (31), which shaft connects together the two fastening elements (31, 32), and which has a cavity which is shaped in such a manner that when assembled it encloses in an enveloping fashion half the surface of the rotary element (34),
**characterised in that**
the oblong shaft (36) comprises a multiplicity of adjacent further rotary elements (35, 35'), and **in that** the cavity of the receiving part (33) is cylindrical in shape, the prosthesis, when assembled, surrounds the rotary element (34) in an annular manner and exerts light pressure on the rotary element (34), wherein the cylindrical cavity forms a through opening in the receiving part (33) through which the rotary elements (34, 35, 35') is pushed before or during the implantation of the prosthesis in the direction of the contact side of the head plate (31) on the eardrum, and the projecting rotary elements can then be nipped off from the further rotary element (35') for cutting to length the shaft (36).

2. The auditory ossicle prosthesis according to Claim 1, **characterised in that** the elastic material of the receiving part (13; 23; 33) is chosen so that its surface has good sliding properties at room or body temperature.

3. The auditory ossicle prosthesis according to Claim 1 or 2, **characterised in that** the rotary element (34) is designed as a sphere or part of a sphere, in particular as a hemisphere.

4. The auditory ossicle prosthesis according to Claim 1 or 2, **characterised in that** the rotary element (34) is designed as a rotary ellipsoid or part of a rotary ellipsoid.

5. The auditory ossicle prosthesis according to one of Claims 1 to 4, **characterised in that** the receiving part (33) is fitted, in particular pressed, into the first fastening element (31).

6. The auditory ossicle prosthesis according to one of Claims 1 to 5, **characterised in that** the receiving part (33) is glued or welded to the first fastening element (31).

7. The auditory ossicle prosthesis according to one of Claims 1 to 4, **characterised in that** the receiving part (33) is a component of the first fastening element (31) designed as a head plate.

8. The auditory ossicle prosthesis according to one of the preceding claims, **characterised in that** the receiving part (33) is produced from silicone or PTFE.

9. The auditory ossicle prosthesis according to one of the preceding claims, **characterised in that** the rotary element (34) and the oblong shaft (36) are produced from metal.

10. The auditory ossicle prosthesis according to one of the preceding claims, **characterised in that** the adjacent further rotary elements (33, 35') of the oblong shaft (36) are shaped identically to the rotary element (34), in particular they have the same outside diameter and are arranged equidistantly along the axis of the shaft (36).

11. The auditory ossicle prosthesis according to one of the preceding claims, **characterised in that** the second fastening element (32) is designed in the shape of a plate, bell, sleeve, piston or as a clip.

12. The auditory ossicle prosthesis according to Claim 11, **characterised in that** the auditory ossicle prosthesis (30) can be fastened on the one hand to the eardrum and on the other hand to the anvil or to the stirrup.

13. The auditory ossicle prosthesis according to one of Claims 1 to 10, **characterised in that** the auditory ossicle prosthesis can be coupled at one end directly to the inner ear, in particular by means of a piston, by opening the human cochlea, which is synonymous with cochleotomy.

14. The auditory ossicle prosthesis according to one of the preceding claims, **characterised in that** the entire auditory ossicle prosthesis (30) or parts thereof are produced from biocompatible plastics, in particular silicone, and/or from fibre composite materials.

15. The auditory ossicle prosthesis according to one of the preceding claims, **characterised in that** parts of the auditory ossicle prosthesis (30) are produced from titanium and/or from gold and/or from tantalum and /or from an alloy of the metals mentioned.

16. The auditory ossicle prosthesis according to one of the preceding claims, **characterised in that** parts of the auditory ossicle prosthesis (30) are produced from a material with a shape memory, which is synonymous with memory effect, in particular from nitinol.

17. The auditory ossicle prosthesis according to one of the preceding claims, **characterised in that** at least one additional mass is fastened to the auditory ossicle prosthesis or to a part of the auditory ossicle chain, in particular by means of a clip, as a function of a desired, predeterminable reverberation of the sound conduction in the middle ear.

18. The auditory ossicle prosthesis according to one of the preceding claims, **characterised in that** the prosthesis is connected to an active vibratory part of an active, in particular implantable, hearing aid

## Revendications

1. Prothèse d'osselet de l'oreille (30) qui remplace ou pallie au moins un élément ou des parties d'un élément de la chaîne des osselets de l'oreille, la prothèse d'osselet de l'oreille (30) présentant au niveau de l'une de ses extrémités, un premier élément de fixation (31) constitué comme une plaque supérieure pour assurer une liaison mécanique avec le tympan et de l'autre de ses extrémités, un deuxième élément de fixation (32) pour assurer une liaison mécanique avec un élément ou des parties d'un élément de la chaîne des osselets de l'oreille, ou avec l'oreille interne et présente entre les deux éléments de fixation (31, 32), une articulation à pivot qui comprend une pièce femelle (33) composée d'une matière plastique élastique qui est reliée fixement au premier élément de fixation (31) dans lequel un élément tournant (34) qui est relié fixement à l'extrémité dirigée vers le premier élément de fixation (31) d'une tige longitudinale (36) qui relie l'un à l'autre les deux éléments de fixation (31, 32) est placé de façon articulée, et qui présente une cavité qui est formée de telle sorte qu'elle comprend en l'entourant, à l'état monté, au moins la demi-surface de l'élément tournant (34),
**caractérisée en ce**
**que** la tige longitudinale (36) comprend une multiplicité d'autres éléments tournants (35, 35') adjacents les uns aux autres et que la cavité de la pièce femelle (33) est de forme cylindrique, entoure l'élément tournant de façon annulaire à l'état monté de la prothèse et exerce une légère pression sur l'élément tournant (34), la cavité cylindrique formant une ouverture constituant un passage dans la pièce femelle (33) par lequel les éléments tournants (34, 35, 35') s'étendent avant ou pendant l'implantation de la prothèse dans le sens du côté d'appui de la plaque supérieure (31) au niveau du tympan et les éléments rotatifs proéminents pouvant être coupés à partir de l'autre élément tournant (35') pour ajuster la longueur de la tige (36).

2. Prothèse d'osselet de l'oreille selon la revendication 1, **caractérisée en ce que** le matériau élastique de la pièce femelle (13 ; 23 ; 33) est choisi de telle sorte que sa surface présente de bonnes propriétés de glissement à température ambiante ou à la température corporelle.

3. Prothèse d'osselet de l'oreille selon la revendication 1 ou 2, **caractérisée en ce que** l'élément tournant (34) est configuré comme une sphère ou une partie d'une sphère, en particulier un hémisphère.

4. Prothèse d'osselet de l'oreille selon la revendication 1 ou 2, **caractérisée en ce que** l'élément tournant (34) est configuré comme un ellipsoïde de rotation ou une partie d'un ellipsoïde de rotation.

5. Prothèse d'osselet de l'oreille selon l'une des revendications 1 à 4, **caractérisée en ce que** la pièce femelle (33) est ajustée, en particulier, enchâssée, dans le premier élément de fixation (31).

6. Prothèse d'osselet de l'oreille selon l'une des revendications 1 à 5, **caractérisée en ce que** la pièce femelle (33) est collée ou soudée au premier élément de fixation (31).

7. Prothèse d'osselet de l'oreille selon l'une des revendications 1 à 4, **caractérisée en ce que** la pièce femelle (33) fait partie intégrante du premier élément de fixation (31) configuré comme une plaque supérieure.

8. Prothèse d'osselet de l'oreille selon l'une des revendications précédentes, **caractérisée en ce que** la pièce femelle (33) est en silicone ou en PTFE.

9. Prothèse d'osselet de l'oreille selon l'une des revendications précédentes, **caractérisée en ce que** l'élément tournant (34) et la tige longitudinale (36) sont en métal.

10. Prothèse d'osselet de l'oreille selon l'une des revendications précédentes, **caractérisée en ce que** les autres éléments tournants (35, 35') adjacents les uns aux autres de la tige longitudinale (36) sont formés de façon identique à l'élément tournant (34), en particulier présentent respectivement le même diamètre extérieur et sont disposés à égale distance le long de l'axe de la tige (36).

11. Prothèse d'osselet de l'oreille selon l'une des revendications précédentes, **caractérisée en ce que** le deuxième élément de fixation (32) est en forme de plaque, en forme de cloche, en forme de gaine, en forme de poinçon ou en forme de clip.

12. Prothèse d'osselet de l'oreille selon la revendication 11, **caractérisée en ce que** la prothèse d'osselet de l'oreille (30) peut être fixée d'une part au niveau du tympan et d'autre part, au niveau de l'enclume ou de l'étrier.

13. Prothèse d'osselet de l'oreille selon l'une des revendications 1 à 10, **caractérisée en ce que** la prothèse d'osselet de l'oreille peut être couplée à l'oreille interne au moyen de l'ouverture de la cochlée humaine, qui a la même signification que la cochléotomie, directement à une extrémité, en particulier par l'intermédiaire d'un piston.

14. Prothèse d'osselet de l'oreille selon l'une des revendications précédentes, **caractérisée en ce que** la prothèse d'osselet de l'oreille complète (30) ou des parties de celle-ci sont fabriquées en matières plastiques biocompatibles, en particulier en silicone, et/ou et en matériaux composites de fibres.

15. Prothèse d'osselet de l'oreille selon l'une des revendications précédentes, **caractérisée en ce que** des parties de la prothèse d'osselet de l'oreille (30) sont fabriquées en titane et/ou en or et/ou en tantale et/ou en un alliage des métaux cités.

16. Prothèse d'osselet de l'oreille selon l'une quelconque des revendications précédentes, **caractérisée en ce que** des parties de la prothèse d'osselet de l'oreille (30) sont fabriquées en un matériau à mémoire de forme, qui a la même signification que « memory effect », en particulier en nitinol.

17. Prothèse d'osselet de l'oreille selon l'une des revendications précédentes, **caractérisée en ce qu'**au moins une masse supplémentaire est fixée en fonction d'une réponse en fréquence souhaitée pouvant être prédéterminée de la conduction acoustique dans l'oreille moyenne au niveau de la prothèse d'osselet de l'oreille ou d'une partie de la chaîne des osselets de l'oreille, en particulier au moyen d'un clip.

18. Prothèse d'osselet de l'oreille selon l'une des revendications précédentes, **caractérisée en ce que** la prothèse est reliée à un élément à vibrations actif d'une prothèse auditive active, en particulier implantable.
